(19) **Europäisches Patentamt / European Patent Office / Office européen des brevets**

(11) **EP 1 767 244 B1**

(12) **FASCICULE DE BREVET EUROPEEN**

(45) Date de publication et mention
de la délivrance du brevet:
**28.10.2009 Bulletin 2009/44**

(51) Int Cl.:
*A61N 1/37* (2006.01)    *A61B 5/0205* (2006.01)
*A61N 1/365* (2006.01)

(21) Numéro de dépôt: **06291503.8**

(22) Date de dépôt: **25.09.2006**

(54) **Dispositif médical implantable actif, notamment dispositif de stimulation, resynchronisation, defibrillation et/ou cardioversion, comprenant des moyens de diagnostic predictif de l'état du patient**

Implantierbare medizinische Vorrichtung, insebesondere zur Stimulation, Resynchronisierung, Defibrillation, und/oder Cardioversion mit diagnostischen MItteln zur Vorhersage des klinischen Zustands des Patienten

Implantable medical device, in particular for stimulation, resynchronisation, defibrillation and/or cardioversion, comprising diagnostic means for predicting the clinical state of the patient

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **27.09.2005 FR 0509837**

(43) Date de publication de la demande:
**28.03.2007 Bulletin 2007/13**

(73) Titulaire: **ELA MEDICAL**
**92541 Montrouge (FR)**

(72) Inventeurs:
• **Casset, Cyrille**
**91130 Ris Orangis (FR)**

• **Torralba, Jean-Mathieu**
**34085 Montpellier (FR)**

(74) Mandataire: **Dupuis-Latour, Dominique et al**
**Bardehle Pagenberg Dost Altenburg Geissler**
**10, boulevard Haussmann**
**75009 Paris (FR)**

(56) Documents cités:
EP-A- 0 750 920     EP-A- 0 804 939
EP-A- 0 966 987     EP-A- 1 317 943
US-A1- 2001 037 067     US-B1- 6 741 885

Printed by Jouve, 75001 PARIS (FR)

## Description

L'invention concerne les « dispositifs médicaux implantables actifs » tels que définis par la directive 90/385/CEE du 20 juin 1990 du Conseil des communautés européennes, notamment les stimulateurs cardiaques, dispositifs de resynchronisation, cardioverteurs et/ou défibrillateurs destinés au traitement des troubles du rythme cardiaque, ou encore les implants actifs à visée purement diagnostique.

Elle concerne plus précisément ces dispositifs dont le fonctionnement est asservi à des paramètres recueillis par des capteurs permettant d'évaluer les besoins métaboliques du porteur de l'appareil ainsi que son niveau courant d'activité.

Ces dispositifs comportent deux capteurs de nature différente, à savoir un capteur de mesure d'un paramètre corporel à prépondérance physiologique et un capteur de mesure d'un paramètre corporel à prépondérance physique. Dans la suite on prendra comme exemple de capteur physiologique un capteur de ventilation-minute (MV), car il s'agit du cas le plus courant, mais ce choix n'a aucun caractère limitatif et d'autres types de capteurs peuvent être également utilisés, produisant un signal représentatif des besoins métaboliques du patient (par exemple un capteur mesurant la saturation d'oxygène dans le sang) ou de son état hémodynamique (par exemple un capteur de bioimpédance intracardiaque). De même, on prendra comme exemple de capteur physique un capteur d'accélération (G), qui est le capteur plus couramment utilisé, mais d'autres types de capteurs sont envisageables pour notamment détecter les mouvements du patient. De façon générale, le capteur physique est caractérisé par un temps de réponse plus court que le capteur physiologique, de manière à permettre une détection très rapide des efforts de courte durée avant même que le paramètre physiologique, de variation plus lente, n'ait évolué de manière significative.

Le EP-A-0 750 920 (ELA Medical) décrit un tel dispositif asservi à deux capteurs, qui opère une sélection de l'un ou l'autre capteur de manière à retenir celui qui donne le signal le plus pertinent à un instant donné. Le EP-A-0 919 255 (ELA Medical) utilise pour l'asservissement une combinaison des signaux délivrés par les deux capteurs.

Outre l'asservissement, les signaux délivrés par les capteurs peuvent être utilisés à des fins de diagnostic de l'insuffisance cardiaque, pour agir de façon appropriée sur la programmation du stimulateur. En particulier, le EP-A-0 966 987 (ELA Medical) permet de suivre l'évolution du patient au cours du temps de manière à donner une représentation adéquate des besoins métaboliques réels de celui-ci, c'est-à-dire prenant en compte le niveau réel d'activité du patient. Le dispositif décrit dans ce document modifie son fonctionnement en cas d'aggravation ou d'amélioration de la situation du patient, par exemple par reprogrammation de certaines de ses fonctions, de manière à suivre l'évolution du patient et s'adapter au degré de décompensation cardiaque effectif du patient.

Le US 2001/037067 A1 décrit également un dispositif dans lequel les informations issues de deux capteurs, physiologique et d'activité, sont cumulées à des fins statistiques pour produire des histogrammes utilisables à des fins de diagnostic.

Le point de départ de l'invention réside dans l'observation de patients qui, même appareillés avec de tels dispositifs perfectionnés, adaptent leur activité quotidienne aux modifications de leur état clinique avec une incidence sur leur niveau d'activité et, dans un certain nombre de cas, sur leur état respiratoire.

En effet, les modifications cliniques pouvant être asymptomatiques, il est courant que le patient adapte inconsciemment son activité à sont état clinique : les premières crises d'insuffisance cardiaque apparaissant à l'effort, ceci conduit le patient à réduire son activité pour éviter la survenue de telles crises.

Les symptômes n'apparaissent plus, le patient ayant modifié son comportement pour les éviter, mais la pathologie continue à progresser. Ainsi, Ce n'est que lorsque l'insuffisance cardiaque gênera le patient même au repos qu'il ira consulter ou, dans les cas extrêmes, devra être hospitalisé en urgence.

En résumé, du fait de cette auto-adaptation, l'absence de symptôme ressenti cause un délai Important entre le début des modifications cliniques et le diagnostic de ces modifications, souvent trop tardif.

L'un des buts de l'invention est de proposer un dispositif du type précité, c'est-à-dire pourvu de deux capteurs physiologique et physique, qui puisse exploiter les informations issues de ces capteurs de manière à évaluer l'état clinique réel du patient et l'évolution de sa pathologie, et ceci même en l'absence de symptômes ressentis, et même si le patient adapte inconsciemment son comportement à l'évolution de son état clinique (c'est-à-dire même s'il réduit son activité pour échapper aux crises d'insuffisance cardiaque).

Un autre but de l'invention est de proposer un dispositif d'aide au diagnostic qui, à partir des données d'état clinique ainsi évaluées, permette d'anticiper l'aggravation de l'insuffisance cardiaque et d'y remédier par un traitement adapté du patient ou une modification du fonctionnement du dispositif.

Un autre but encore de l'invention est de proposer un tel dispositif qui puisse détecter une aggravation brusque de l'état clinique du patient même en l'absence de symptôme grave, et délivrer un signal d'alarme préventif permettant de prendre au plus vite les mesures appropriées et éviter ainsi le déclenchement soudain d'une crise à brève échéance.

L'état clinique du patient est reflété sous forme d'un ou plusieurs indices d'état clinique, mis à jour par exemple quotidiennement par le dispositif.

Le diagnostic prédictif, quant à lui, se base sur l'évolution de ces indices sur le long terme, en analysant les variations de ceux-ci sur une période de plusieurs jours. Cette analyse peut être effectuée quotidiennement, en interne par le dispositif ou bien avec l'aide d'un équipement externe, par exemple pour afficher les différents index et présenter les résultats sur un écran pour évaluation par un praticien.

Le dispositif de l'invention est du type à double capteur tel que décrit notamment dans le US 2001/037067 A1 précité, correspondant au préambule de la revendication 1. L'invention comprend les éléments énoncés à la partie caractérisante de cette revendication. les sous-revendications présentent des formes de mise en oeuvre avantageuses.

On va maintenant décrire plus en détail un exemple de mise en oeuvre de l'invention, en référence aux dessins annexés.

La figure 1 est un schéma par blocs montrant les différentes fonctions du dispositif impliquées dans la détermination des index d'état clinique et dans le diagnostic prédictif.

La figure 2 illustre la manière dont sont prises en compte les variations du signal d'accélération par le dispositif de l'invention.

La figure 3 illustre les variations de la ventilation-minute moyenne en activité, sur une période de plusieurs semaines.

La figure 4 illustre la fonction de transfert pour la détermination des index d'état clinique relatifs à l'accélération et au temps passé en activité.

La figure 5 illustre la fonction de transfert pour la détermination des index d'état clinique relatifs à la ventilation-minute en activité et au repos.

La figure 6 est un exemple d'affichage des divers index d'état clinique et méta-index de diagnostic déterminés par le dispositif de l'invention.

On va maintenant décrire un exemple de réalisation du dispositif de l'invention. En ce qui concerne ses aspects logiciels, l'invention peut être mise en oeuvre par une programmation appropriée du logiciel de commande d'un stimulateur asservi connu.

L'invention peut notamment être appliquée aux dispositifs implantables commercialisés par ELA Médical, Montrouge, France tels que les appareils *Symphony* et *ELA Rhapsody*. Il s'agit de dispositifs à microprocesseur programmables comportant des circuits pour recevoir, mettre en forme et traiter des signaux électriques recueillis par des électrodes et divers capteurs. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

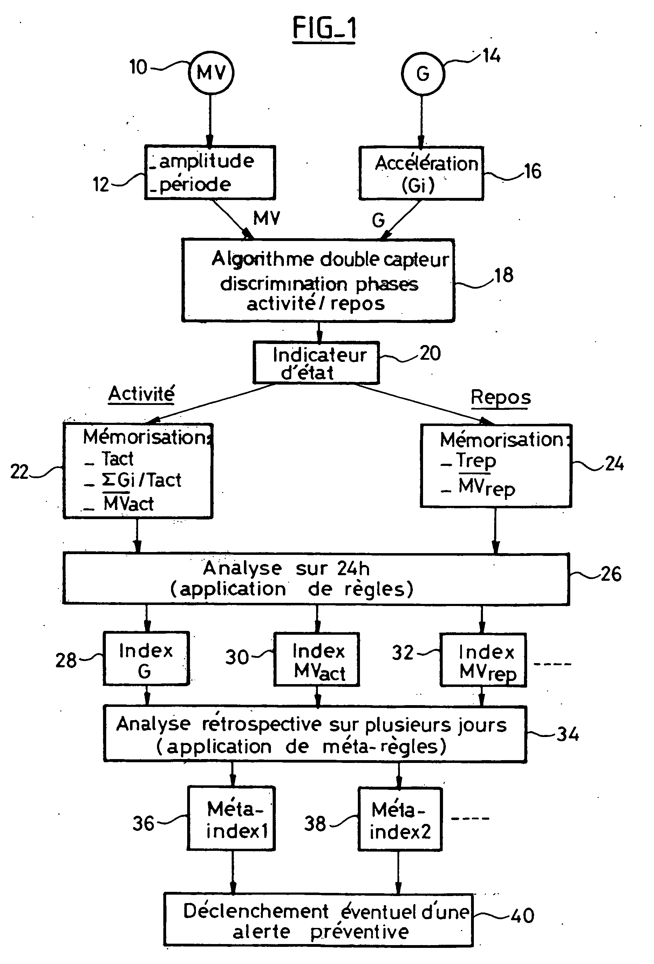

Comme illustré figure 1, le dispositif comporte un capteur 10 délivrant un signal représentatif des besoins métaboliques du patient, typiquement un signal d'impédance transthoracique dont l'analyse des variations périodiques (amplitudes et périodes successives) est opérée par le bloc 12 qui délivre un signal de ventilation-minute (MV). Le dispositif comporte également un capteur physique permettant de détecter les mouvements du patient, typiquement un capteur d'accélération 14 associé à un circuit d'échantillonnage 16 délivrant une succession d'échantillons numérisés $G_i$, avec un pas $i = 125$ ms par exemple.

À partir des informations MV et G délivrées concurremment, le dispositif opère un asservissement de type « double capteur » (bloc 18) comme décrit dans les EP-A-0 750 920 et EP-A-0 919 255 antérieurs précités, notamment un asservissement de la fréquence de stimulation et une adaptation éventuelle des paramètres de fonctionnement. Cette fonction d'asservissement proprement dite n'entre toutefois pas dans le cadre de l'invention et ne sera pas décrite en détail.

L'algorithme d'asservissement présente toutefois l'avantage de posséder une fonction de discrimination des phases d'activité et de repos du patient (bloc 18) à partir des indications instantanées par les capteurs MV et G, dont il résulte un indicateur d'état 20 pouvant prendre au moins deux valeurs « activité » et « repos » (d'autres valeurs étant également possibles, par exemple « récupération après effort » qui sera assimilé à une phase d'activité, ou « sommeil » qui n'est qu'un cas particulier de phase de repos).

L'invention propose de mémoriser dans une mémoire du dispositif, de façon distincte pour les phases d'activité et pour les phases de repos, les données fournies par les capteurs MV et G.

Plus précisément, le dispositif comporte une première mémoire 22 regroupant les paramètres caractéristiques en phase d'activité, notamment :

- le temps passé en activité sur les dernières 24 heures (Tact),
- la somme des mesures du capteur G en phase d'activité, pondérée sur les dernières 24 heures ($\Sigma G_i$/Tact), et
- la ventilation-minute moyenne (MVact) pendant les phases d'activité, mesurée sur les dernières 24 heures.

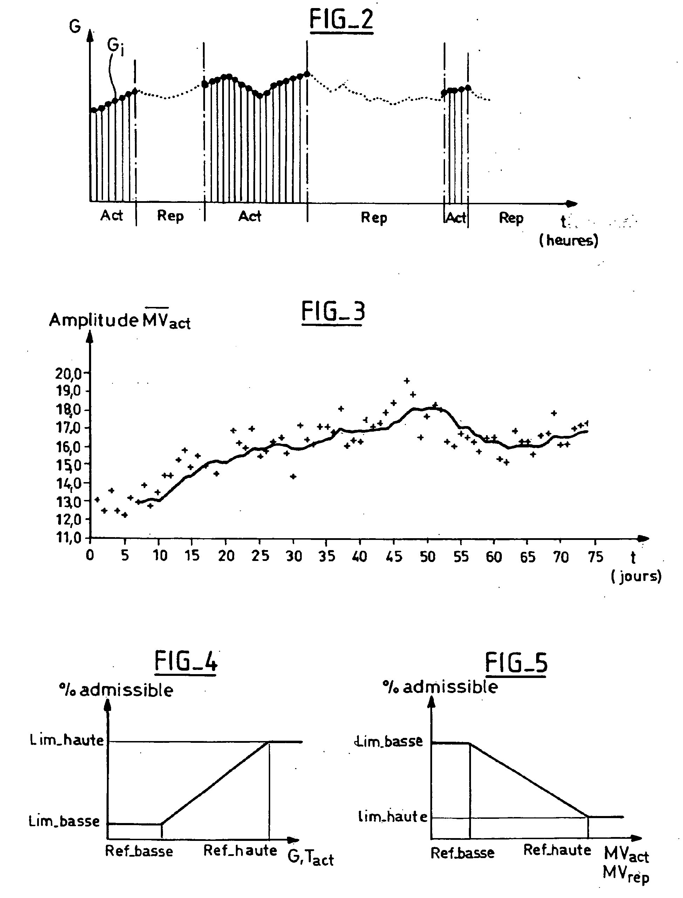

La figure 2 illustre plus précisément la manière dont est obtenue et mise à jour la donnée $\Sigma G_i$.

Comme on l'a indiqué plus haut, le capteur G fournit une série d'échantillons numérisés $G_i$ à intervalles de par exemple i = 125 ms, dont la variation au fil du temps est illustrée sur la figure 2. Par ailleurs, l'indicateur d'état 20 permet de distinguer les périodes d'activité (Act) et de repos (Rep).

Le dispositif procède à la sommation des valeurs $G_i$ des échantillons depuis les dernières 24 heures, mais en inhibant cette sommation pendant les périodes de repos, en ne sommant donc que des valeurs correspondant à des périodes d'activité. Le total obtenu (sommation sur les dernières 24 heures) est divisé, pour pondération, par la durée Tact qui est le temps passé en activité.

Le paramètre MVact est une valeur moyenne de la ventilation-minute MV lors des périodes d'activité.

La figure 3 illustre par exemple les variations de ce paramètre MVact au cours du temps sur une durée de plusieurs semaines : les croix indiquent les valeurs calculées quotidiennement et mémorisées dans la mémoire 22, le trait continu indiquant une moyenne mobile sur 7 jours.

Le dispositif comporte également une seconde mémoire 24 regroupant les paramètres caractéristiques en phase de repos, notamment :

- le temps passé en repos sur les dernières 24 heures (Trep), et
- la ventilation-minute moyenne (MVrep) pendant les phases de repos, mesurée sur les dernières 24 heures.

Le paramètre MVrep est une valeur moyenne de la ventilation-minute MV lors des périodes de repos.

Ces diverses informations mémorisées et mises à jour dans les deux tableaux de valeurs 22 et 24 sont ensuite soumis à une analyse (bloc 26) consistant à appliquer un certain nombre de règles d'inférence donnant une série d'index cliniques, tel qu'un index 28 lié à l'accélération (index G), un index 30 lié à la ventilation-minute en activité (index MVact), un index 32 lié à la ventilation-minute au repos (index MVrep), etc.

Cette analyse est opérée par exemple chaque jour, avec mise à jour quotidienne des divers index d'état clinique.

Plusieurs méthodes d'analyse sont possibles. Une première méthode consiste à comparer les valeurs mémorisées à diverses références fixes, et à détecter le franchissement de seuils hauts et/ou bas.

Une autre méthode avantageuse, que l'on va décrire ci-dessous, consiste à analyser les variations des données d'un jour au suivant (ou sur un autre intervalle, par exemple d'une semaine à la suivante, etc.) et comparer cette variation à une valeur de référence correspondant à un pourcentage de variation physiologiquement admissible compte tenu de l'état clinique du patient. Le franchissement de cette limite révèlera la dégradation de l'état du patient à l'égard du critère correspondant (activité, ventilation basale, etc.)

On va décrire en détail par exemple la règle d'inférence correspondant au critère G, les règles correspondant aux autres critères étant appliquées de façon comparable.

La mémoire 22 contenant les paramètres relatifs aux phases d'activité contient une série de valeurs *Somme_ G(i),* le pas *i* correspondant ici à un intervalle d'une journée.

Plutôt que d'appliquer la règle d'inférence à la valeur quotidienne isolée, on calcule préalablement une moyenne mobile sur une semaine de manière à pondérer les variations d'activité d'un jour à l'autre, moyenne que l'on peut exprimer de la manière suivante :

$$M7G(i) = (1/7) * \sum_{k=i-6}^{i} somme\_G(k)$$

La règle relative au paramètre G sera désignée "règle R1", et le résultat de son application sera un indicateur pouvant prendre trois valeurs, représentatives de l'évolution de l'état clinique du patient au regard de ce paramètre G : +1 (amélioration), -1 (dégradation), ou 0 (stabilité).

En effet, l'augmentation de l'activité est un élément favorable dans le tableau clinique du patient, ce qu justifie le score +1, tandis qu'une réduction de ses activités est au contraire un élément défavorable sur le long terme.

Ces valeurs sont attribuées de la manière suivante :

Règle R1 :

Si M7G(i) > M7G(i-6)*$\alpha$(M7G(i)), alors R1(i) = +1 ;
Si M7G(i) < M7G(i-6)*$\alpha$(M7G(i)), alors R1(i) = -1 ;
R1(i) = 0 sinon

$\alpha$ (M7G(i)) étant un pourcentage calculé à partir d'une fonction de transfert dépendant de la valeur courante du critère considéré.

L'intérêt d'un facteur $\alpha$ non constant est lié au besoin de tenir compte des résultats précédents pour qualifier l'amélioration ou la dégradation du résultat : ainsi, pour un patient qui a une activité très faible une augmentation d'activité, même modeste, sera un élément très favorable, ce qui n'est pas nécessairement le cas pour un patient ayant une activité soutenue et en augmentation régulière.

La figure 4 illustre la fonction de transfert donnant le taux d'augmentation admissible (ci-après *Seuilparamètre*) en fonction de la valeur du paramètre considéré.

Dans l'exemple indiqué plus haut du paramètre G, la fonction de transfert est définie de la manière suivante :

*Seuilparamètre*(M7(i)) = *Limite_basse* si M7(i) < *Réf_basse* ;
*Seuilparamètre*(M7(i)) = *Limite_haute* si M7(i) > *Réf haute* ;
*Seuilparamètre*(M7(i)) = A*M7(i)+B,

A, B étant la pente et l'ordonnée à l'origine de la droite passant par les points (*Réf_basse, Limite_basse*) et (*Réf_haute, Limite_haute*).

Le principe de cette fonction de transfert est également applicable, *mutatis mutandis,* à la durée passée en activité Tact.

En revanche, pour le paramètre de ventilation, en activité (MVact) ou au repos (MVrep), le sens de variation de la fonction de transfert doit être inversé, comme illustré sur la figure 5. En effet, dans le cas où un patient est à une ventilation de repos élevée, la plus petite augmentation suffit à le gêner fortement, alors qu'à plus faible ventilation, il possède encore une marge d'augmentation importante.

Les résultats ainsi obtenus par l'application des diverses règles peuvent faire l'objet d'un diagnostic plus poussé, par application de métarègles, permettant de définir un ou plusieurs index de niveau supérieur, ou « méta-index ».

Ces méta-index sont déterminés (bloc 34) par une analyse rétrospective des valeurs successives prises par les différents index.

Ces métarègles peuvent être élaborées de diverses manières, et on en donnera ci-dessous simplement un exemple, consistant à définir une limite sur plusieurs jours (*p* jours) sur lesquels il faut qu'une règle R délivre un nombre minimum de valeurs positives de l'index correspondant (ce minimum étant paramétré à la valeur *Seuil_MR*) :

Métarègle MR (une valeur +1 du méta-index étant représentative d'une aggravation significative de l'état du patient) :

$$MR(i) = \sum_{k=i-p+1}^{i} R(k), \text{ avec p entier naturel}$$

Si MR(i) > *Seuil_MR,* alors MR(i) = +1 ;
MR(i) = 0 sinon

Cette métarègle prend en compte le fait d'avoir des valeurs d'index nulles ou négatives: en effet, des valeurs nulles n'augmentent pas la valeur MR(i) du méta-index, tandis que des valeurs négatives ont pour effet de « compenser » les valeurs positives précédemment prises.

On obtient ainsi un ou plusieurs méta-index 36, 38, ... reflétant l'évolution de l'un des paramètres G, MVact, MVrep, ... ou d'une combinaison de ces divers paramètres.

Le tout peut être visualisé et affiché, par exemple pour permettre à un praticien d'appréhender rapidement l'évolution de l'état clinique du patient et estimer les risques d'aggravation soudaine de cet état.

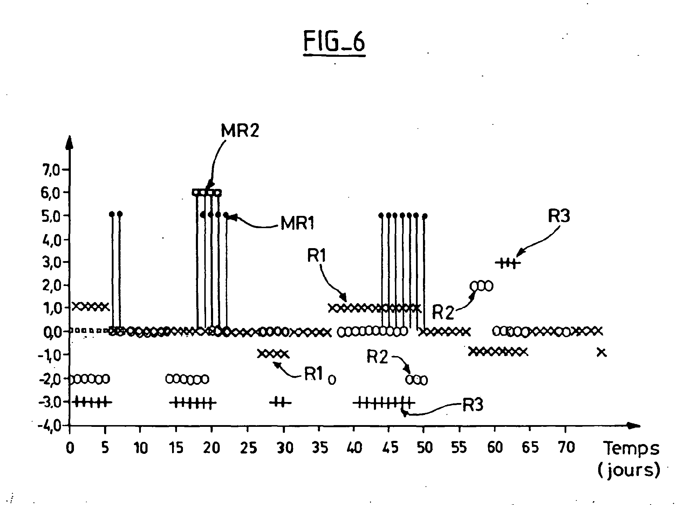

On a représenté sur la figure 6 un exemple d'un tel affichage.

Chaque unité d'abscisse correspond à un jour, et l'on a affiché les valeurs déterminées chaque jour pour l'index relatif à l'activité G (résultant de l'application de la règle R1 et représenté par un « X »), pour l'index de ventilation en activité MVact (résultant de l'application de la règle R2 et représenté par un « O ») et pour l'index de ventilation au repos MVrep (résultant de l'application de la règle R3 et représenté par un « + »).

Pour la clarté de l'affichage, et pour éviter tout risque de superposition, on a placé à l'abscisse ±1 le marqueur correspondant à une valeur ±1 de la règle R1, à l'abscisse ±2 le marqueur correspondant à une valeur ±1 de la règle R2 et à l'abscisse ±3 le marqueur correspondant à la valeur ±1 de la règle R3.

On peut voir sur l'exemple illustré que la règle 2, concernant la ventilation en activité, indique une aggravation de l'état du patient (augmentation de la ventilation en activité) aux dates 14 ... 19, 37, et 48 ... 50, une amélioration de l'état (baisse de la ventilation en activité) aux dates 57 ... 59, et un état stationnaire partout ailleurs. Ces résultats sont à rapprocher de ceux illustrés figure 3, qui présentent la variation du paramètre MVact jour après jour.

L'affichage donne également les résultats des métarègles, avec les méta-index MR1 et MR2 : on a placé à l'abscisse +4 le marqueur correspondant à une valeur +1 de la métarègle R1 et à l'abscisse +5 le marqueur correspondant à une valeur +1 de la métarègle R2. Le méta-index MR1 résulte par exemple de l'application d'une métarègle sur la ventilation au repos, tandis que le méta-index MR2 résulte de l'application d'une métarègle combinant niveau d'activité et ventilation en activité.

Ainsi,aux dates 44 ... 50, le méta-index MR1 signale une aggravation de l'état lié à la ventilation au repos, du fait que l'index R3 a pris la valeur -1 pendant au moins cinq jours sur sept.

Des alarmes peuvent être déclenchées en fonction des résultats produits par les métarègles (et également par les règles).

Ces alarmes permettent d'anticiper l'apparition d'un évènement tel qu'une crise d'insuffisance cardiaque, afin de prendre sans attendre les mesures nécessaires pour empêcher la survenue de cette crise ou, à tout le moins, en réduire les effets. Ce diagnostic peut être ainsi opéré de façon extrêmement précoce, avant tout symptôme grave et donc bien avant que le patient n'ait fait appel de lui-même à son praticien ou à un service d'urgence.

**Revendications**

1. Un dispositif médical implantable actif, notamment un dispositif de stimulation, resynchronisation, défibrillation et/ou cardioversion, ou un implant actif à visée diagnostique, comprenant :

   - des moyens (10, 12) de mesure d'un paramètre corporel à prépondérance physiologique, notamment la ventilation-minute (MV), délivrant un signal physiologique,
   - des moyens (14, 16) de mesure d'un paramètre corporel à prépondérance physique, notamment l'accélération (G), délivrant un signal physique,
   - des moyens (18) discriminateurs des phases d'activité et de repos, opérant en réponse aux signaux physiologique et physique et délivrant un indicateur d'état du patient (20),
   - une mémoire contenant une pluralité de champs, et
   - des moyens de traitement statistique, aptes à recevoir en entrée les signaux physiologique et physique et l'indicateur d'état, à délivrer en sortie des informations traitées et à mettre à jour sélectivement lesdits champs à partir de ces informations traitées,
   dispositif **caractérisé en ce que :**
   - lesdits champs comprennent :

      un premier tableau de valeurs (22) regroupant des données caractéristiques des phases d'activité du patient, et
      un second tableau de valeurs (24) regroupant des données caractéristiques des phases de repos du patient,

   - le dispositif est apte à mémoriser, de façon distincte pour les phases d'activité et pour les phases de repos, les données délivrées par lesdits moyens de mesure d'un paramètre corporel à prépondérance physiologique et par lesdits moyens de mesure d'un paramètre corporel à prépondérance physique,
   - les moyens de traitement statistique mettent à jour de manière dissociée le premier et le second tableau de valeurs, sélectivement en fonction de la valeur prise par l'indicateur d'état, et
   - le dispositif comprend en outre des moyens d'analyse (26), aptes à évaluer au moins un index d'état clinique (28, 30, 32) à partir des données contenues dans les champs à la fois du premier et du second tableau de valeurs.

2. Le dispositif de la revendication 1, dans lequel:

   - le premier tableau de valeurs (22) comprend des champs représentatifs de la durée écoulée en activité (Tact) et du niveau des signaux physiologique (MV) et physique (G) pendant cette durée écoulée en activité, et
   - le second tableau de valeurs (24) comprend des champs représentatifs de la durée écoulée en repos (Trep) et du niveau du signal physiologique (MV) pendant cette durée écoulée en repos.

3. Le dispositif de la revendication 2, dans lequel le premier tableau de valeurs contient un cumul ($\Sigma G_i$) des valeurs numérisées successives du signal physique sur une durée prédéterminée, ce cumul n'étant opéré que lorsque l'indicateur d'état indique une phase d'activité du patient.

4. Le dispositif de la revendication 2, dans lequel le premier et le second tableau de valeurs contiennent chacune une moyenne respective (MVact ; MVrep) des valeurs numérisées successives du signal physiologique sur une durée prédéterminée, cette moyenne n'étant mise à jour que lorsque l'indicateur d'état indique une phase, respectivement d'activité ou de repos, du patient.

**5.** Le dispositif de la revendication 1. dans lequel les moyens d'analyse comprennent des moyens de comparaison d'au moins certaines desdites données par rapport à une valeur de référence.

**6.** Le dispositif de la revendication 1, dans lequel les moyens d'analyse comprennent des moyens de comparaison des variations d'au moins certaines desdites données sur une période prédéterminée par rapport à une valeur de référence.

**7.** Le dispositif de la revendication 5 ou 6, dans lequel ladite valeur de référence est une valeur calculée, fonction du niveau moyen pris par la donnée correspondante sur une période prédéterminée.

**8.** Le dispositif de la revendication 7, dans lequel la variation de ladite valeur de référence calculée est bornée par des limites haute et/ou basse.

**9.** Le dispositif de la revendication 1, dans lequel les moyens d'analyse sont également aptes à évaluer au moins un méta-index (36, 38) à partir des valeurs successives prises par l'index d'état clinique.

**10.** Le dispositif de la revendication 9, dans lequel le dispositif comprend en outre des moyens d'alerte préventive (40), aptes à signaler le franchissement d'un seuil par au moins l'un desdits méta-index d'état clinique évalués par les moyens d'analyse.

**11.** Le dispositif de la revendication 10, dans lequel lesdits index et méta-index d'état clinique sont réévalués à une périodicité quotidienne.

**Claims**

**1.** An active implantable medical device, particularly a device for stimulation, resynchronisation, defibrillation and/or cardioversion, or an active implant for diagnostic purposes, said device comprising:

- means (10,12) for measuring a predominantly physiologic corporal parameter, in particular minute-ventilation (MV) which provide a physiological signal,
- means (14, 16) for measuring a predominantly physical corporal parameter, in particular acceleration (G), which provide a physical signal,
- means (18) for discriminating rest and activity phases, operating in response to said physiologic and physical signals, which provide a patient status index (20),
- a memory containing a plurality of fields, and
- means for statistical treatment, adapted to receive as an input said physiologic and physical signals and said status index, to provide as an output said treated information and to selectively update said fields from said treated information,

wherein said device is **characterized in that** :

- said fields comprise :

    - a first array of values (22) summarizing data characteristic of patient's activity phases, and
    - a second array of values (24) summarizing data characteristic of patient's rest phases,

- said device is adapted to store, separately for activity and rest phases, the data provided by said means for measuring a predominantly physiological corporal parameter and by said means for measuring a predominantly physical corporal parameter,
- said means for statistical treatment separately update said first and second arrays of values, selectively as a function of the value taken by said status index, and
- said device furthermore comprises analysis means (26) adapted to evaluate at least one clinical status index (28, 30, 32) from the data contained in the fields both of the first and the second arrays of values.

**2.** Device according to claim 1, wherein :

- the first array of values (22) comprises fields representative of the elapsed duration of activity (Tact) and of the level of physiologic and physical signals over that elapsed duration of activity, and
- the second array of values (24) comprises fields representative of the elapsed duration of rest (Trep) and of the level of physiologic signal (MV) over that elapses duration of rest.

3. Device according to claim 2, wherein the first array of values contains a sum ($\Sigma G_i$) of successive digitized values of the physical signal over a predetermined duration, said sum being operated only when the status index shows a patient's activity phase.

4. Device according to claim 2, wherein the first and the second arrays of values each contain respectively an average (MVact ; MVrep) of the successive digitized values of the physiologic signal over a predetermined duration, said average being updated only when the status index indicates a patient's activity or rest phase respectively.

5. Device according to claim 1, wherein the analysis means comprise a comparison means for comparing at least some of said data to a reference value.

6. Device according to claim 1, wherein the analysis means comprise means for comparing variations of at least some of said data over a predetermined duration to reference value.

7. Device according to claim 5 or 6, wherein said reference value is a calculated value, that in a function of the average level of corresponding data over a predetermined duration.

8. Device according to claim 7, wherein said variation of said calculated reference value is limited by high and/or low limits.

9. Device according to claim 1, wherein the analysis means are also adapted to evaluate at least one metaindex (36, 38) from the successive values taken by the clinical status index.

10. Device according to claim 9, wherein said device furthermore comprises preventive alarm means (40 adapted to report passing of a threshold through at least on of said clinical status metaindexes that are evaluated by the analysis means.

11. Device according to claim 10, wherein said clinical status indexes and metaindexes are reevaluated on a daily basis.

**Patentansprüche**

1. Eine aktive implantierbare medizinische Vorrichtung, insbesondere eine Vorrichtung zur Stimulation, Resynchronisation, Defibrillation und/oder Kardioversion oder ein aktives Implantat zur diagnostischen Absicht, umfassen :

   - ein physiologisches Signal liefernde Mittel (10, 12) zur Messung eines körperlichen, im wesentlichen physiologischen Parameters, insbesondere der Ventilation/Minute (MV),
   - ein physisches Signal liefernde Mittel (14,16) zur Messung eines körperlichen, im wesentlichen physischen Parameters, insbesondere der Beschleunigung (G)
   - in Beantwortung vom physiologischen bzw. physischen Signal arbeitende, einen Zustandsindex (20) des Patienten liefernde, die Aktivitäts- und Ruhephase diskriminierende Mittel (18),
   - einen Datenspeicher enthaltend eine Vielzahl von Felder, und
   - Mittel zur statistischen Verarbeitung, die geeignet sind, das physiologische bzw. physische Signal und den Zustandsindex als Eingabe zu empfangen, die verarbeiteten Informationen als Ausgang zu liefern und besagte Felder ab jenen verarbeiteten Informationen zu aktualisieren,

   **dadurch gekennzeichnet, dass**

   - besagte Felder

      - eine erste, die für die Aktivitätsphase des Patienten charakteristischen Daten zusammenfassende Tabelle von Werten (22),
      - eine zweite, die für die Ruhephase des Patienten charakteristischen Daten zusammenfassende Tabelle von Werten (24) umfassen,

- die Vorrichtung geeignet ist, die durch obengenannte Mittel zur Messung eines körperlichen, im wesentlichen physiologischen Parameters bzw. durch obengenannte Mittel zur Messung eines körperlichen, im wesentlichen physischen Parameters gelieferte Daten getrennt für die Aktivitätsphase und für die Ruhephase zu speichern,
- die Mittel zur statistischen Verarbeitung die erste bzw. die zweite Tabelle von Werten selektiv in Abhängigkeit von der vom Zustandsindex genomm Wert aktualisieren,
- die Vorrichtung zusätzlich Analysemittel (26) umfasst, die geeignet sind, wenigstens einen klinischen Zustandsindex (28, 30, 32) ab den in den Feldern sowohl der ersten als der zweiten Tabelle von Werten enthaltenden Daten abzuschätzen.

2.  Die Vorrichtung gemäss Anspruch 1, wobei

    - die erste Tabelle von Werten (22) Felder umfasst, die von der in Aktivität abgelaufenen Zeitdauer (Tact) und der Höhe der physiologischen (MV) bzw. physischen (G) Signale während dieser in Aktivität abgelaufenen Zeitdauer charakteristisch sind,
    - die zweite Tabelle von Werten (24) Felder umfasst, die von der in Ruhe abgelaufenen Zeitdauer (Trep) und der Höhe des physiologischen Signals (MV) während dieser in Ruhe abgelaufenen Zeitdauer charakteristisch sind.

3.  Die Vorrichtung gemäss Anspruch 2, wobei die erste Tabelle von Werten eine Gesamtsumme ($\Sigma G_i$) der aufeinanderfolgenden digitalisierten Werte des physischen Signals während einer vorbestimmten Zeitdauer umfasst, wobei diese Gesamtsumme erst dann ermittelt wird, wenn der Zustandsindex eine Aktivitätsphase des Patienten anzeigt.

4.  Die Vorrichtung gemäss Anspruch 2, wobei die erste und die zweite Tabelle von Werten je einen respektiven Mittelwert (MVact ; MVrep) der aufeinanderfolgenden digitalisierten Werte des physiologischen Signals während einer vorbestimmten Zeitdauer enthält, wobei dieser Mittelwert erst dann aktualisiert wird, wenn der Zustandsindex eine Aktivitäts- bzw. Ruhephase des Patienten anzeigt.

5.  Die Vorrichtung gemäss Anspruch 1, wobei die Analysemittel Vergleichsmittel aufweisen, die die Veränderungen wenigstens einiger besagter Daten mit einem Bezugswert vergleichen.

6.  Die Vorrichtung gemäss Anspruch 1, wobei die Analysemittel Vergleichsmittel aufweisen, die die Veränderungen wenigstens einiger besagter Daten während einer vorbestimmten Laufzeit mit einem Bezugswert vergleichen.

7.  Die Vorrichtung gemäss Anspruch 5 oder 6, wobei besagter Bezugswert ein kalkulierter Wert ist, der vom Durchschnittswert der entsprechenden Daten während einer vorbestimmten Zeitdauer abhängt.

8.  Die Vorrichtung gemäss Anspruch 7, wobei die Veränderung besagtes kalkulierten Bezugswerts durch niedrige bzw. hohe Grenzen begrenzt ist.

9.  Die Vorrichtung gemäss Anspruch 1, wobei die Analysemittel auch geeignet sind, wenigstens einen Metaindex (36, 38) ab den aufeinanderfolgenden Werten des klinischen Zustandsindexes abzuschätzen.

10. Die Vorrichtung gemäss Anspruch 9, wobei die Vorrichtung zusätzlich vorbeugende Alarmmittel (40) umfasst, die geeignet sind, das Überschreiten einer Schwelle dank wenigstens eines besagter durch die Analysemittel abgeschätzten klinischen Zustandsmetaindexe erkennen zu lassen.

11. Die Vorrichtung gemäss Anspruch 10, wobei besagte klinischen Zustandsindexe und -metaindexe mit einer täglichen Periodizität neu abgeschätzt werden.

## FIG_1

## FIG_2

G

G$_i$

Act | Rep | Act | Rep | Act | Rep | t (heures)

## FIG_3

Amplitude $\overline{MV}_{act}$

20,0
19,0
18,0
17,0
16,0
15,0
14,0
13,0
12,0
11,0

0  5  10  15  20  25  30  35  40  45  50  55  60  65  70  75  t (jours)

## FIG_4

% admissible

Lim_haute

Lim_basse

Ref_basse    Ref_haute    $G, T_{act}$

## FIG_5

% admissible

Lim_basse

lim_haute

Ref_basse    Ref_haute    $MV_{act}$ $MV_{rep}$

## FIG_6

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0750920 A, ELA Medical
- EP 0919255 A, ELA Medical
- EP 0966987 A
- US 2001037067 A1